# FASCICULE DE BREVET EUROPEEN

(11) **EP 4 380 997 B1**
(45) Date de publication et mention de la délivrance du brevet: **14.10.2026**
(21) Numéro de dépôt: 22758233.5
(22) Date de dépôt: 01.08.2022
(51) Int. Cl.: C08G 63/78, C08G 63/08

(54) **PROCÉDÉ DE RÉCUPÉRATION DE LACTIDE ET D'ACIDE LACTIQUE LORS DES ÉTAPES DE PRODUCTION DE POLYLACTIDE (PLA)**
VERFAHREN ZUR RÜCKGEWINNUNG VON LACTID UND MILCHSÄURE WÄHREND POLYLACTID (PLA)-HERSTELLUNGSSCHRITTEN
METHOD FOR RECOVERING LACTIDE AND LACTIC ACID DURING POLYLACTIDE (PLA) PRODUCTION STEPS

(30) Priorité: 02.08.2021 BE 202100054
(43) Date de publication de la demande: 12.06.2024
(73) Titulaire: Futerro S.A., 7760 Escanaffles (BE)
(72) Inventeur: COSZACH, Philippe, 7760 Escanaffles (BE); VAN GANSBERGHE, Martin, 7769 Escanaffles (BE)
(74) Mandataire: AWA Benelux
(86) Numéro de dépôt international: PCT/EP2022/071579
(87) Numéro de publication internationale: WO 2023/012118

(56) Documents cités:
- EP-B1- 2 675 799
- EP-B1- 3 080 187
- JP-A- H10 101 777

## Description

### Domaine de l'invention

La présente invention se rapporte à un procédé de production de polylactide (PLA), et en particulier à un procédé amélioré qui permet de récupérer un maximum de matières utiles afin de recycler sans perte et donc d'améliorer le rendement global du processus de production de PLA à partir de l'acide lactique. Plus spécifiquement encore la présente invention se rapporte à un procédé permettant d'apporter une solution à la problématique du recyclage des flux gazeux contenant du lactide.

### Etat de l'art

On sait que parmi les procédés usuels de production de PLA il en existe un premier type qui consiste en une polycondensation directe de l'acide lactique, comme décrit dans le brevet JP 733861 ; mais ce type de procédé est limité par l'utilisation d'un solvant et donc par les difficultés à éliminer l'eau hors du milieu réactionnel. Un autre type de procédé usuel de production de PLA consiste à partir d'acide lactique selon un procédé comprenant plusieurs étapes qui consistent à oligomériser l'acide lactique en oligomères, cycliser les oligomères en lactide, purifier le lactide brut et polymériser le lactide purifié en PLA avant de démonomériser et dévolatiliser le PLA obtenu afin d'en extraire le lactide non-converti avant l'ultime étape qui comprend la granulation, la cristallisation et le séchage des granules de PLA. EP2675799B1 divulgue un procédé de fabrication d'un acide polylactique dans lequel le rendement est augmenté par rapport au produit final par recyclage du lactide à partir d'un courant secondaire issu de la purification de l'acide polylactique brut et recyclage du lactide obtenu issu de cette purification.

Tout au long de ces étapes des procédés de préparation de PLA, toute une série de flux secondaires sont générés et conduisent à des pertes de matière conséquentes comme lors des purges et soutirages des vapeurs qui sont essentiellement à pression réduite, de l'ordre de 0,1 mbar à 400 mbar et plus spécifiquement de 1 mbar à 200 mbar, et qui contiennent notamment de l'eau, de l'acide lactique, du lactide et autres petits oligomères d'acide lactique ou d'esters d'acide lactique en différentes compositions.

On a également constaté que selon ces procédés usuels, en prenant comme référence un processus théorique sans aucun recyclage, le rendement global est de l'ordre de 50% molaire.

Il a déjà été envisagé d'améliorer ce rendement en contrôlant le recyclage, dit facile, de certains flux comme par exemple au niveau des condensats d'oligomérisation, ou de la purification du lactide, ce qui permet de déjà faire remonter le rendement global à environ 75%. Cependant, ces rendements restent insuffisants pour conduire un procédé industriel.

La Demanderesse a déjà proposé pour améliorer le rendement global du procédé, d'agir au niveau de la purification des sous-produits des étapes de purification du lactide par une distillation pour en extraire un mélange de lactide purifié, le flux ainsi généré étant recyclé dans le flux principal pour réduire les pertes et moduler la teneur en énantiomère -D- du PLA produit comme décrit dans le brevet WO2014/180836. La Demanderesse a également proposé de récupérer un maximum d'unités lactates des flux secondaires par un procédé d'estérification/trans-estérification/distillation et hydrolyse de l'ester en acide lactique comme décrit dans le brevet WO2015/086613.

D'autre part, et comme cela a été exposé plus avant, une part non négligeable des purges et soutirages s'effectue sous forme vapeur, et il est également bien connu dans l'état de l'art que l'on peut récupérer ces vapeurs pour les reconvertir en lactide d'abord et en acide lactique ensuite par simple hydrolyse.

On peut donc récupérer les vapeurs des produits à chacune des étapes du procédé, et en premier lieu les soumettre à une condensation, qui s'effectue le plus souvent de manière indirecte par passage du courant gazeux sur des surfaces froides.

On connaît en outre le brevet US 5266706 qui décrit un procédé pour récupérer le lactide d'un courant gazeux contenant entre autres de l'acide lactique et de l'eau, par lavage de ce courant gazeux avec un solvant non miscible à l'eau, comme les hydrocarbures non-polaires ou cycloaliphatiques, ou encore halogénés. Selon ce procédé on règle la température pendant le lavage de manière à récupérer l'eau, le lactide et l'acide lactique. Cependant une ultime étape devra nécessairement être consacrée à séparer le solvant.

Afin de pallier les problèmes générés par la présence du solvant, on peut citer le brevet US 8430948 qui montre une autre manière de traiter le courant gazeux contenant du lactide, de l'acide lactique et de l'eau. Selon ce procédé, le courant de vapeur est transféré par une conduite pour alimenter une colonne de condensation, et y est récupéré dans le récipient situé au fond de cette colonne. Il y constitue de la sorte une phase liquide qui contient du lactide dissous, de l'acide lactique ainsi que l'eau de condensation et de lavage.

L'acide lactique présent dans le fond de cette colonne de condensation est pompé via un échangeur, pour être renvoyé vers l'appareil de distribution de la colonne de condensation.

L'acide lactique tombe ensuite sur un empilage et se mélange au courant gazeux provenant du procédé.

Le procédé décrit dans le brevet US 8430948 est ajusté pour qu'il y ait dissolution du lactide dans le fond de la colonne, sans que la teneur en lactide dissous dans le milieu liquide (eau + acide lactique et lactide) ne dépasse 5% en poids.

Les non-condensables sont évacués en tête de colonne par la conduite vers le groupe de vide.

En outre, la notion de condensation du lactide présent dans la phase vapeur est également décrite dans le brevet FR 2726559 qui montre un procédé de traitement de ce flux en phase vapeur contenant entre autres le lactide. Il comprend un entraînement à la vapeur d'eau, la condensation des vapeurs de lactide, d'acide lactique et d'eau et la formation d'une solution aqueuse dans laquelle du lactide se dissout et précipite.

On peut encore citer à cet effet le brevet US 9700840 qui se rapporte plus particulièrement au traitement de la solution aqueuse après condensation de la phase vapeur selon un procédé qui consiste à soumettre cette solution aqueuse successivement à un chauffage, une réaction et un refroidissement, avec pour strictes conditions d'empêcher tout apport extérieur d'acide lactique dans le système et toute précipitation de lactide dans le circuit.

On voit que si des solutions existent pour assurer le recyclage du lactide en acide lactique aux différentes étapes du procédé de préparation de PLA, ces solutions comportent des conditions opératoires qui peuvent freiner la récupération du lactide et de l'acide lactique pour pouvoir les réutiliser. On citera notamment l'ajustement de la dissolution du lactide ou encore l'exclusion de précipitation du lactide dans la solution aqueuse, ce qui a pour conséquence de limiter le rendement global de la production de PLA, et de constituer un inconvénient majeur pour un procédé industriel.

Il existe donc un besoin pour mettre en œuvre un procédé permettant de recycler sans contrainte les flux secondaires de lactide et d'acide lactique issus des différentes étapes du procédé de préparation de PLA et tout en permettant un rendement global très élevé, et ce sans impacter la flexibilité du procédé.

### Résumé de l'invention

La Demanderesse a maintenant trouvé un procédé qui permet de réduire les inconvénients cités ci-dessus, ce procédé consistant à :
- Amener, ensemble ou séparément, les divers flux gazeux contenant du lactide, de l'acide lactique et de l'eau
- Envoyer ce flux gazeux sur un ensemble de colonnes à empilage comprenant au moins une colonne, où il sera condensé.
- Mettre en contact ce flux gazeux dans cet ensemble de colonnes avec un fluide de mouillage à une température comprise entre 1 et 30°C, ce fluide comprenant de l'eau, de l'acide lactique et du lactide dissous
- Récupérer le fluide sortant de l'ensemble de colonnes à empilage à une température comprise entre 15°C et 70°C et préférentiellement entre 20 et 60°C, ce fluide contenant entre autres du lactide sous forme solide à une teneur comprise entre 0.1 et 20% en poids, par rapport au poids total de lactates présents en solution, préférentiellement entre 1 et 10% en poids et plus préférentiellement entre 2 et 5% en poids par rapport au poids total de lactates présents en solution.
- Optionnellement filtrer et extraire au moins partiellement le lactide précipité présent dans ce fluide
- Réchauffer cette solution aqueuse qui peut être partiellement débarrassée du lactide solide via au moins un échangeur pour amener la température à une valeur comprise entre 50 et 95°C et préférentiellement entre 60 et 80°C.
- Envoyer cette solution aqueuse dans une cuve agitée et alimentée en continu par un courant d'acide lactique, cette cuve, située en amont du présent circuit de traitement, étant maintenue à une température entre 50 et 95°C et préférentiellement entre 60 et 80°C.
- Réaliser dans cette cuve l'hydrolyse du lactide présent dans la solution aqueuse, afin de le transformer en acide lactique

Il est bien entendu que selon le procédé de l'invention on récupère de l'acide lactique et/ou des oligomères d'acide lactique plus spécifiquement des dimères d'acide lactique, mais celui-ci peut également se trouver sous forme d'ester d'acide lactique comme le lactate de méthyle, d'éthyle, de butyle ou autres.

### Description détaillée de l'invention

Le procédé de la présente invention est également décrit au moyen des figures suivantes, mais ne doit pas être interprétée comme limitée à celles-ci :
La Figure 1 schématise le procédé de traitement des divers flux gazeux provenant par exemple de l'oligomérisation, de la cyclisation, de la purification du lactide et de la dévolatilisation du PLA qui sont soit collectés en un seul flux, soit traités séparément pour être soumis à une série d'opérations afin de récupérer de l'acide lactique ou oligomère léger d'acide lactique, ce traitement comprenant au minimum une colonne à empilage (12), surmontée d'un groupe de vide (20).
La Figure 2 schématise une mise en œuvre particulière du même procédé mais où les colonnes à empilage (12) et (14) sont connectées en parallèle.
La Figure 3 schématise une autre mise en œuvre particulière du même procédé mais où les colonnes à empilage (12) et (14) sont connectées en série.

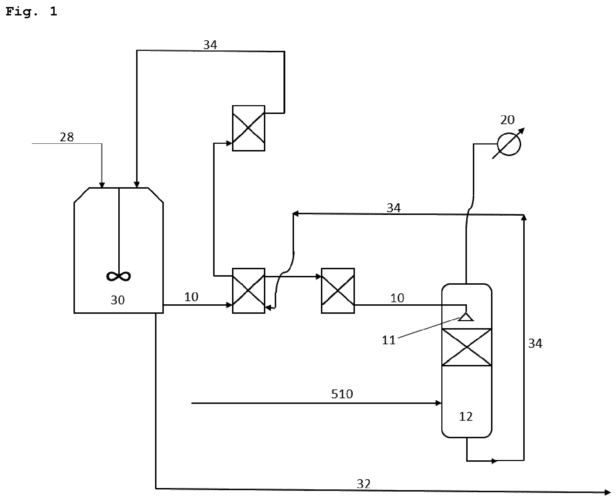

Selon la Figure 1, on décrit un procédé qui consiste à traiter les divers flux gazeux contenant du lactide, de l'acide lactique et de l'eau en les faisant passer ensemble ou séparément par la conduite (510) afin de les amener à l'alimentation de la colonne à empilage (12) ; le niveau de l'alimentation à cette colonne (12) est situé en-dessous du distributeur (11) ; la colonne à empilage (12) est également alimentée avec la solution aqueuse acide provenant de la cuve (30) via la conduite (10) et ayant traversé une série d'échangeurs pour baisser sa température à une valeur comprise entre 1 et 30°C préférentiellement 1 et 20°C, de préférence entre 5 et 15°C, avant de la distribuer dans la colonne (12) via le distributeur (11). On soutire ensuite le pied de colonne (12) sous forme d'une solution aqueuse contenant de l'acide lactique, de l'eau, du lactide dissous et du lactide précipité, cette solution, dont la température est comprise entre 15 et 70°C, étant recyclée, via au moins un échangeur (deux échangeurs ou plus peuvent être envisagés pour optimiser les récupérations énergétiques) afin de porter la température à une valeur comprise entre 50 et 95°C, vers la cuve (30), via la conduite (34). La cuve (30) est, en plus de la solution aqueuse de recyclage, alimentée en continu par une solution d'acide lactique via la conduite (28) ; l'hydrolyse du lactide en acide lactique ou oligomère léger d'acide lactique a lieu dans cette cuve (30). D'autre part, on soutire de cette cuve (30) une partie de la solution aqueuse acide pour l'envoyer à l'étape d'évaporation via la conduite (32) afin de récupérer l'acide lactique concentré, tandis qu'une autre partie alimente via la conduite (10) la colonne à empilage (12).

La solution aqueuse circulant entre la colonne à empilage (12) et la cuve (30) contient du lactide précipité à une teneur comprise entre **0.1** et 20% en poids par rapport au poids total de lactates présents en solution.

Selon ce procédé, on peut également lors du soutirage du pied de la colonne (12) filtrer, en mode batch ou continu, la solution pour récupérer partiellement le lactide précipité (non représenté sur la figure 1).

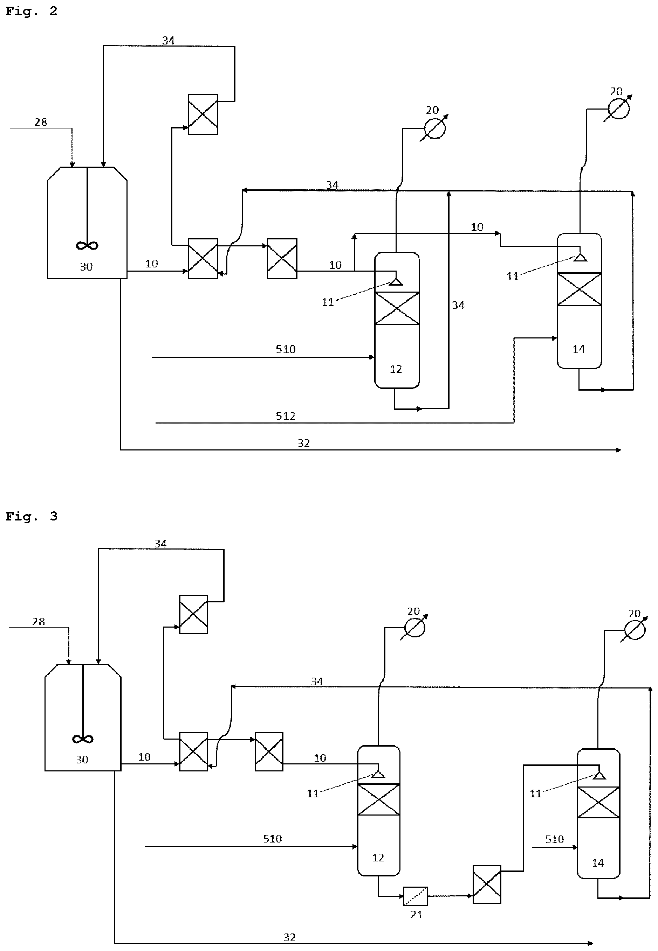

Selon la mise en œuvre particulière du procédé décrite à la Figure 2 on a simplement ajouté une colonne à empilage (14) à la première colonne (12) pour le traitement des flux gazeux, ces colonnes étant disposées en parallèle. Elles sont alimentées par les flux gazeux contenant du lactide, de l'acide lactique et de l'eau, via les conduites (510) et (512) et par la solution aqueuse acide provenant de la cuve (30) via la conduite (10).

On soutire une solution aqueuse contenant de l'acide lactique, de l'eau, du lactide dissous et du lactide précipité aux pieds des colonnes (12) et (14) pour l'envoyer vers au moins un échangeur et de préférence deux échangeurs et porter la température à une valeur comprise entre 50 et 95°C, préférentiellement entre 55 et 85°C, avant d'être recyclée à la cuve (30) via la conduite (34), qui est, en plus de cette solution aqueuse acide, alimentée en continu par une solution d'acide lactique via la conduite (28). Les lactides présents dans cette cuve (30) sont hydrolysés en acide lactique et/ou oligomère d'acide lactique. On retrouve entre les pieds des colonnes (12) et (14) et la cuve (30), du lactide solide en une quantité de 0.1 à 20% en poids par rapport au poids total de lactates présents en solution et préférentiellement de 1 à 10% en poids par rapport au poids total de lactates présents en solution, en plus du lactide dissous. D'autre part, on soutire de cette cuve (30) une partie de la solution aqueuse acide pour l'envoyer à l'étape d'évaporation via la conduite (32) afin de récupérer l'acide lactique concentré, tandis qu'une autre partie alimente via la conduite (10) les colonnes à empilage (12) et (14).

Selon la mise en œuvre particulière du procédé décrite à la Figure 3 on effectue le traitement du flux gazeux au moyen de deux colonnes à empilage connectées ici en série. Selon cette configuration du dispositif, une opération de filtration (via filtre 21) et d'extraction partielle du lactide précipité provenant du soutirage du pied de la colonne à empilage (12), en mode batch ou continu, s'avère nécessaire voire indispensable. En mode batch, il est également possible de mettre deux filtres en parallèle afin de permettre un travail en continu. Cependant, la solution aqueuse circulant entre la colonne à empilage (14) et la cuve (30) contient nécessairement du lactide précipité à une teneur comprise entre 0.1 et 20% en poids par rapport au poids total de lactates présents en solution.

On soutire la solution aqueuse contenant de l'acide lactique, de l'eau, du lactide dissous et du lactide précipité de la seconde colonne (14) pour l'envoyer vers au moins un échangeur, pour porter sa température à une valeur comprise entre 50 et 95°C, préférentiellement entre 60 et 75°C, avant retour à la cuve (30), qui en plus de cette solution aqueuse acide est alimentée en continu via la conduite (28) par une solution d'acide lactique. Les lactides présents dans cette cuve (30) sont hydrolysés en acide lactique et/ou oligomère d'acide lactique ; d'autre part, on soutire de cette cuve (30) une partie de la solution aqueuse acide pour l'envoyer à l'étape d'évaporation via la conduite (32) afin de récupérer l'acide lactique concentré, tandis qu'une autre partie alimente via la conduite (10) la colonne à empilage (12).

On montre ainsi que le procédé de l'invention permet de récupérer, sans blocage du circuit, un maximum de flux secondaires tout en évitant les conditions drastiques pour la récupération du lactide comme enseigné dans les brevets de l'état de l'art.

Le procédé de la présente invention permet donc de récupérer plus de flux secondaires afin de pouvoir les recycler presque complètement dans le processus de préparation de PLA ou dans d'autres opérations, sans perte aucune.

Selon un mode d'exécution du procédé de l'invention, on peut traiter ensuite l'acide lactique récupéré dans la cuve (30) d'abord par une pré-concentration effectuée sur un évaporateur à multiples effets suivi d'une concentration sur couche mince et enfin une distillation conformément au procédé décrit dans le brevet BE 1011197 pour obtenir un acide lactique de qualité « polymer grade ».

L'acide lactique « polymer grade » récupéré peut alors être recyclé dans le procédé de production de PLA décrit dans le brevet WO2015/086613 (par exemple au niveau de l'oligomérisation), et ainsi assurer des rendements élevés en réduisant les pertes.

Le procédé de la présente invention est également décrit et illustré à l'aide des exemples ci-dessous, mais qui n'en constituent pas une limitation.

### Exemple 1 :

On a préparé du PLA selon le procédé décrit dans le brevet WO2015/086613 de la Demanderesse. Ce procédé prévoit déjà certains recyclages mais pas selon le procédé de la présente invention.

On a alors collecté les flux secondaires en phase vapeur, comme décrit à la Figure 3, ceux-ci contenant du lactide, de l'acide lactique et de l'eau, on les a rassemblés dans la conduite (510), et on les a envoyés vers les colonnes à empilage (12) et (14) connectées en série. Ces flux sont alors condensés et mis en contact avec une solution aqueuse acide provenant de la cuve (30), amenée via la conduite (10) au distributeur (11) de la colonne à empilage (12). La solution aqueuse récoltée en pied de la colonne (12) est filtrée, afin d'éliminer partiellement le lactide solide,55% du lactide solide présent a ainsi été retiré. La solution aqueuse est ensuite, après avoir été refroidie dans un échangeur, envoyée vers la colonne à empilage (14) pour être distribuée par le distributeur (11), elle est également mise en contact avec une autre partie du flux gazeux (510) (contenant de l'eau, de l'acide lactique et du lactide).

On soutire la solution aqueuse contenant de l'acide lactique, du lactide dissous et du lactide précipité en plus de l'eau, du pied de la seconde colonne (14) (réchauffée à 20°C), pour l'envoyer vers deux échangeurs, le premier étant destiné à de la récupération énergétique, pour porter sa température à 70°C avant de la recycler à la cuve (30), qui en plus de cette solution aqueuse acide est alimentée en continu via la conduite (28) par une solution d'acide lactique frais. Les lactides présents dans cette cuve (30) sont hydrolysés en acide lactique. Dans la partie de circuit comprise entre le pied de la colonne (14) et la cuve (30), il y a présence de lactide solide à une teneur de 2,4% en poids, par rapport au poids total de lactates présents en solution, en plus du lactide dissous et de l'acide lactique frais ajouté.

On a ensuite traité l'acide lactique récupéré dans la cuve (30) en le soumettant à une concentration sur un évaporateur à multiples effets suivi d'un passage sur un évaporateur sur couche mince et enfin d'une distillation, conformément au procédé décrit dans le brevet BE1011197 pour obtenir un acide lactique de qualité « polymer grade ». L'acide lactique « polymer grade » récupéré est alors recyclé sans aucune contrainte au niveau de l'étape d'oligomérisation du procédé de production de PLA décrit dans le brevet WO2015/086613. Le rendement Lactide/PLA obtenu est supérieur, bien que proche, à celui de ce brevet mais permet surtout de valoriser tous les recyclages ce qui limite les pertes de manière générale.

### Exemple 2

Comme dans l'exemple 1, on a préparé du PLA selon le procédé décrit dans le brevet WO2015/086613 de la Demanderesse. On a repris les flux secondaires en phase vapeur, comme décrit à la Figure 2, ceux-ci contenant du lactide, de l'acide lactique et de l'eau, on les a rassemblés dans les conduites (510) et (512), et on les a envoyés vers les colonnes à empilage (12) et (14) connectées en parallèle. Ces flux sont alors condensés et mis en contact avec une solution aqueuse acide provenant de la cuve (30), amenée via la conduite (10) au distributeur (11) des colonnes à empilage (12) et (14).

On soutire la solution aqueuse contenant de l'acide lactique, du lactide dissous et un peu de lactide précipité (à une teneur de respectivement 1,4% et 1.6% en poids par rapport au poids total des lactates présents en solution)en plus de l'eau, du pied des colonnes (12) et (14), pour l'envoyer vers deux échangeurs, le premier étant destiné à de la récupération énergétique, pour finalement porter sa température à 70°C avant de la recycler à la cuve (30) via la conduite (34), qui en plus de cette solution aqueuse acide est alimentée en continu via la conduite (28) par une solution d'acide lactique frais. Les lactides présents dans cette cuve (30) sont hydrolysés en acide lactique et/ou oligomère d'acide lactique.

On a ensuite traité l'acide lactique récupéré dans la cuve (30) en le soumettant d'abord à une concentration sur un évaporateur à multiples effets suivi d'un passage sur un évaporateur sur couche mince et enfin d'une distillation, conformément au procédé décrit dans le brevet BE1011197 pour obtenir un acide lactique de qualité « polymer grade ».

## Revendications

1. Procédé à rendement global amélioré de préparation de PLA comprenant les étapes suivantes et qui consiste à :
- amener, ensemble ou séparément, les divers flux gazeux contenant du lactide, de l'acide lactique et de l'eau,
- envoyer ce flux gazeux sur un ensemble de colonnes à empilage comprenant au moins une colonne, où il sera condensé,
- mettre en contact ce flux gazeux dans cet ensemble de colonnes avec un fluide de mouillage à une température comprise entre 1 et 30°C, ce fluide comprenant de l'eau, de l'acide lactique et du lactide dissous,
- récupérer le fluide sortant de l'ensemble de colonnes à empilage à une température comprise entre 15°C et 70°C, ce fluide contenant entre autres du lactide sous forme solide à une teneur comprise entre 0.1 et 20% en poids par rapport au poids total de lactates présents dans la solution,
- optionnellement filtrer et extraire au moins partiellement le lactide précipité présent dans ce fluide,
- réchauffer cette solution aqueuse qui peut être partiellement débarrassée du lactide solide via au moins un échangeur pour amener la température à une valeur comprise entre 50 et 95°C,
- envoyer cette solution aqueuse acide dans une cuve agitée et alimentée en continu par un courant d'acide lactique, cette cuve, située en amont du présent circuit de traitement, étant maintenue à une température entre 50 et 95°C, et
- réaliser dans cette cuve l'hydrolyse du lactide présent dans la solution aqueuse, afin de le transformer en acide lactique et/ou oligomère d'acide lactique.

2. Procédé selon la revendication 1, **caractérisé en ce que** les colonnes à empilage sont connectées en parallèle.

3. Procédé selon la revendication 1, **caractérisé en ce que** les colonnes à empilage sont connectées en série.

4. Procédé selon la revendication 3, **caractérisé en ce que** l'on filtre et l'on extrait au pied de la première colonne à empilage au moins partiellement le lactide précipité.

5. Procédé selon les revendications 1 à 4, **caractérisé en ce que** le fluide de mouillage a de préférence une température de 5 à 12 °C.

6. Procédé selon les revendications 1 à 5, **caractérisé en ce que** le fluide sortant de l'ensemble de colonnes à empilage a de préférence une température comprise entre 20°C et 60°C.

7. Procédé selon les revendications 1 à 6, **caractérisé en ce que** la solution aqueuse partiellement débarrassée du lactide solide est réchauffée à une température comprise préférentiellement entre 55 et 85°C et plus préférentiellement entre 60 et 75°C.

8. Procédé selon l'une quelconque des revendication 1 à 7, **caractérisé en ce que** la solution aqueuse acide circulant entre la colonne à empilage (12) et/ou (14) et la cuve (30) contient du lactide sous forme précipitée.

9. Procédé selon la revendication 8, **caractérisé en ce que** la solution aqueuse contient du lactide sous forme précipitée de préférence à une teneur de 1 à 10% en poids par rapport au poids de lactates présents dans la solution.

10. Procédé selon l'une quelconque des revendications 1 à 9, **caractérisé en ce que** l'acide lactique récupéré après hydrolyse dans la cuve (30) est soumis à une concentration comprenant au moins un évaporateur sur couche mince et à une distillation pour obtenir de l'acide lactique de qualité « polymer grade ».

## Patentansprüche

1. Verbesserter Gesamtdurchsatzprozess zur Vorbereitung von PLA, umfassend die folgenden Schritten und bestehend aus:
- die verschiedenen Gasströme, das Laktid, Milchsäure und Wasser enthalten, gemeinsam oder getrennt zuzuführen,
- diesen Gasstrom zu einer Reihe von stapelbaren Säulen zu leiten, die mindestens eine Säule umfassen, in der er kondensiert wird,
- diesen Gasstrom in dieser Reihe von Säulen mit einer Benetzungsflüssigkeit bei einer Temperatur zwischen 1 und 30°C, wobei die Flüssigkiet Wasser, Milchsäure und gelöstes Laktid enthält, in Kontakt zu bringen,
- die aus der Reihe von stapelbaren Säulen austretende Flüssigkeit bei einer Temperatur zwischen 15°C und 70°C zurückzugewinnen, wobei diese Flüssigkeit unter anderem Laktid in fester Form in einem Gehalt zwischen 0,1 und 20 Gew.-% bezogen auf das Gesamtgewicht der in der Lösung vorhandenen Laktate enthält,
- optional filtern und zumindest teilweise das in dieser Flüssigkeit vorhandene ausgefällte Laktid extrahieren,
- diese wässrige Lösung, aus der das feste Laktid über mindestens einen Austauscher entfernt werden kann, erhitzen um die Temperatur auf einen Wert zwischen 50 und 95°C zu bringen,
- diese saure wässrige Lösung in einen Rührbehälter leiten, der kontinuierlich mit einem Milchsäurestrom versorgt wird; dieser Rührbehälter, der sich stromaufwärts des aktuellen Behandlungskreislaufs befindet, wird bei einer Temperatur zwischen 50 und 95°C gehalten, und
- Durchführung der Hydrolyse des in der wässrigen Lösung vorhandenen Laktids in diesem Rührbehälter, um es in Milchsäure und/oder Milchsäure-Oligomer umzuwandeln.

2. Verfahren nach Anspruch 1, **dadurch gekennzeichnet, dass** die stapelbaren Säulen parallel verbunden sind.

3. Verfahren nach Anspruch 1, **dadurch gekennzeichnet, dass** die stapelbaren Säulen in Reihe geschaltet sind.

4. Verfahren nach Anspruch 3, **dadurch gekennzeichnet, dass** das ausgefällte Laktid am Fuß der ersten stapelbaren Säule gefiltert und zumindest teilweise extrahiert wird.

5. Verfahren nach den Ansprüchen 1 bis 4, **dadurch gekennzeichnet , dass** die Benetzungsflüssigkeit vorzugsweise eine Temperatur von 5 bis 12 °C hat.

6. Verfahren nach den Ansprüchen 1 bis 5, **dadurch gekennzeichnet, dass** die aus der stapelbaren Säulenreihe austretende Flüssigkeit vorzugsweise eine Temperatur zwischen 20°C und 60°C hat.

7. Verfahren nach den Ansprüchen 1 bis 6, **dadurch gekennzeichnet, dass** die wässrige Lösung, aus der das feste Laktid teilweise entfernt wurde, auf eine Temperatur von vorzugsweise zwischen 55 und 85 °C und noch bevorzugter zwischen 60 und 75 °C erhitzt wird.

8. Verfahren nach einem der Ansprüche 1 bis 7, **dadurch gekennzeichnet, dass** die saure wässrige Lösung, die zwischen den stapelbaren Säulen (12) und/oder (14) und dem Rührbehälter (30) zirkuliert, Laktid in ausgefällter Form enthält.

9. Verfahren nach Anspruch 8, **dadurch gekennzeichnet, dass** die wässrige Lösung Laktid in ausgefällter Form enthält, vorzugsweise mit einem Gehalt von 1 bis 10 Gew.-% im Verhältnis zum Gewicht der in der Lösung vorhandenen Laktate.

10. Verfahren nach irgendeinem der Ansprüche 1 bis 9, **dadurch gekennzeichnet, dass** die nach der Hydrolyse im Rührbehälter gewonnene Milchsäure (30) einer Konzentration, die mindestens einen Dünnschichtverdampfer umfasst, und einer Destillation unterzogen wird um Milchsäure in "Polymer-Grade"-Qualität zu gewinnen.

## Claims

1. A PLA preparation process with improved overall yield, comprising the following steps and which consists of:
- Providing, together or separately, the various gas streams comprising lactide, lactic acid and water;
- Sending this gas stream to an assembly of packed columns comprising at least one column, where it will be condensed;
- Bringing this gas stream in contact with a wetting fluid in this assembly of columns at a temperature between 1 and 30°C, this fluid comprising water, lactic acid and dissolved lactide;
- Recovering the fluid leaving the assembly of packed columns at a temperature between l5°C and 70°C, this fluid *inter alia* comprising lactide in solid form in an amount between 0.1 and 20% by weight, relative to the total weight of lactates present in the solution;
- Optionally, filtering and extracting at least partially the precipitated lactide present in this fluid;
- Re-heating this aqueous solution, from which the solid lactide may have been partially removed via at least one exchanger to bring the temperature to a value between 50 and 95°C;
- Sending this acidic aqueous solution to a tank which is stirred and continuously fed by a stream of lactic acid, wherein this tank, located upstream of the present treatment circuit, is maintained at a temperature between 50 and 95°C, and
- Hydrolysis of the lactide present in the aqueous solution in this tank for conversion thereof into lactic acid and/or lactic acid oligomer.

2. Method according to claim 1, wherein the packed columns are connected in parallel.

3. Method according to claim 1, wherein the packed columns are connected in series.

4. Method according to claim 3, wherein the precipitated lactide is filtered and at least partially extracted at the foot of the first packed column.

5. Method according to claims 1 to 4, wherein the wetting fluid preferably has a temperature between 5 and 12 °C.

6. Method according to claims 1 to 5, wherein the fluid leaving the assembly of packed columns preferably has a temperature between 20°C and 60°C.

7. Method according to claims 1 to 6, wherein the aqueous solution, from which the solid lactide has been partially removed, is reheated to a temperature preferably between 55 and 85°C, and more preferably between 60 and 75°C.

8. Method according to any one of claims 1 to 7, wherein the acidic aqueous solution circulating between the packed column (12) and/or (14) and the tank (30) comprises lactide in precipitated form.

9. Method according to claim 8, wherein the aqueous solution comprises lactide in precipitated form preferably in an amount between 1 and 10% by weight, relative to the weight of lactates present in the solution.

10. Method according to any one of claims 1 to 9, wherein the lactic acid recovered after hydrolysis in the tank (30) is subjected to concentration comprising at least one thin-film evaporator, and to distillation to obtain lactic acid of polymer grade quality.
